# EUROPEAN PATENT APPLICATION

(11) **EP 4 372 754 A1**
(43) Date of publication of application: **22.05.2024**
(21) Application number: 22208000.4
(22) Date of filing: 17.11.2022
(51) Int. Cl.: G16H 20/30, G16H 30/20, G16H 30/40, G16H 40/63, G16H 50/20, G16H 50/70

(54) **A SYSTEM FOR ASSESSING DENTAL PROFESSIONAL POSTURE**

(71) Applicant: Koninklijke Philips N.V., 5656 AG Eindhoven (NL)
(72) Inventor: RMAILE, Amir Hussein, Eindhoven (NL); PERRONE, Antonio Luigi, Eindhoven (NL); KOOIJMAN, Gerben, Eindhoven (NL); SPELT, Hanne Adriana Alijda, Eindhoven (NL); GERHARDT, Lutz Christian, Eindhoven (NL); JOHNSON, Mark Thomas, Eindhoven (NL)
(74) Representative: Philips Intellectual Property & Standards

(57) **Abstract**

The present invention provides a computer-implemented method for assessing dental professional posture, a system for carrying out the claimed method. The method comprises receiving, from a sensor, dental procedure plan information. The dental procedure plan information indicates a dental procedure to be carried out by the dental professional. A predicted posture is determined based on the dental procedure plan information. A posture guidance recommendation is generated based on the posture information and the predicted posture. The method further comprises outputting the posture guidance recommendation.

## Description

### FIELD OF THE INVENTION

This invention relates to a method and system for assessing the posture of a dental professional.

### BACKGROUND OF THE INVENTION

Dental professionals are often required to spend a significant amount of time carrying out procedures that involve awkward working postures and repetitive tasks. Additionally, dental procedures performed by the dental professional may require the dental professional to stand for prolonged periods. Repeated exposure to this type of work can result in damage to muscles, joints, bones, ligaments, tendons, nerves and blood vessels, which can lead to pain, fatigue and musculoskeletal disorders. As a result, dental professionals are at high risk for neck and/or back pain. The type of pain varies, ranging from a stiff feeling to a more definite, localized pain. Lower back pain is the most frequent complaint, and most dental professionals worldwide have experienced this during their careers. It is thought that 80% of dental professionals suffer from neck and back pain throughout their career. Patients treated by a rested, comfortable dental professional will benefit from an improved patient experience and may benefit from better health outcomes.

It would be desirable to support dental professionals to work in a healthier way, thereby improving health outcomes for both dental professionals and their patients.

### SUMMARY OF THE INVENTION

The invention is defined by the claims.

According to examples in accordance with an aspect of the invention, there is provided a computer-implemented method for assessing dental professional posture, the method comprising:
receiving, from a sensor, posture information;
receiving dental procedure plan information, the dental procedure plan information indicating a dental procedure to be carried out by the dental professional;
determining a predicted posture based on the dental procedure plan information; and
generating and outputting a posture guidance recommendation based on the posture information and the predicted posture.

Information received from a sensor/array of sensors arranged to sense dental professional posture is used, in combination with dental procedure plan information, to provide a recommended posture to the dental professional.

The posture information may comprise information indicating a posture adopted by a dental professional and optionally information indicating the duration over which the dental professional maintained that posture. The posture information is obtained using a posture sensor. The posture sensor may provide posture information by detecting whether and how dental equipment is being used. Alternatively, the posture sensor may monitor posture in a different way (e.g., via use of a wearable sensor).

The predicted posture is determined based on dental procedure plan information. Based on the predicted posture and the posture information, an algorithm generates and outputs a posture guidance recommendation to support the dental professional in making adjustments to their posture, which may be beneficial to their comfort and/or long-term health.

The computer implemented method may further comprise:
determining a posture duration associated with the predicted posture, wherein the posture guidance recommendation is further based on the posture duration.

Posture duration, which is the amount of time which the dental professional is expected to maintain the predicted posture, is determined. Based on the posture information, the predicted posture and the determined posture duration, an algorithm generates and outputs a posture guidance recommendation to support the dental professional in making adjustments to their posture.

Determining a posture duration may comprise determining an amount of time associated with the predicted posture based on the dental procedure plan information, or predicting an amount of time associated with the predicted posture. Accordingly, the posture duration may be predicted based on the dental procedure plan information.

Determining a predicted posture may comprise:
running a prediction algorithm configured to:
receive, as input variables, posture information and dental procedure plan information identifying a procedure to be performed by the dental professional, and
determine, as output, based on the input variables, a predicted posture, and preferably wherein the prediction algorithm is further configured to determine, as output, a posture duration.

The prediction algorithm may be configured to determine a predicted posture based on input dental procedure plan information and to determine a posture duration associated with the predicted posture using input posture information. That is, information about a previous posture can be used to predict the duration of a future posture.

Alternatively, the posture duration may be predicted, for example based on the procedure type and/or historical procedure information.

The prediction algorithm may be configured to determine a predicted posture based on a combination of posture information received from at least one dental equipment sensor and dental procedure plan information. The prediction algorithm may be a trained machine learning algorithm trained on a dataset comprising information obtained from the at least one (dental equipment) sensor and dental procedure plan information.

The computer implemented method may further comprise:
receiving, as an input variable to the prediction algorithm, physiological information and/or historical procedure information.

The physiological patient information may indicate the height of the patient, the height of the dental professional and/or the expected patient pain level. The prediction algorithm may use physiological information and/or historical procedure information as inputs when determining the predicted posture and/or predicted posture duration.

The computer implemented method may further comprise predicting, based on the predicted posture and the dental procedure plan information, a subsequent predicted posture, wherein predicting the subsequent predicted posture comprises:
running a prediction algorithm configured to:
receive, as an input variable, an initial predicted posture and a procedure identifier, and
determine, as output, based on the input variables, a subsequent predicted posture.

Accordingly, the prediction algorithm may predict a sequence of postures associated with a dental procedure based on the predicted posture and the procedure identified by the procedure plan. The prediction algorithm may be a trained machine learning algorithm.

The computer implemented method may further comprise predicting, based on the dental procedure plan information, a subsequent posture duration associated with the subsequent predicted posture, wherein the prediction algorithm is configured to:
receive, as input variables, an initial predicted posture and a procedure identifier, and
determine, as output, based on the input variables, a subsequent posture duration.

Accordingly, the prediction algorithm may predict a duration for each pose in a sequence of postures associated with a dental procedure based on the initial predicted posture and the procedure identified by the procedure plan.

Generating and outputting a posture guidance recommendation may comprise running a recommendation algorithm configured to:
receive, as input variables, the posture information, the predicted posture and posture duration, and
determine, as output, based on the input variables, a recommended posture or a recommendation to avoid a posture,
and optionally wherein the method further comprises determining, as output, at least one dental procedure compatible with the recommended posture or at least one dental procedure associated with the posture it is recommended to avoid.

Recommending a posture to adopt, or avoid, can support a dental professional in determining which postures and procedures would mitigate, or exacerbate, issues associated with spending excessive time in a particular posture. The recommendation algorithm may be a trained machine learning algorithm.

Recommending a procedure/procedures to avoid, can support a dental professional in determining which procedures, when performed by the dental professional, would mitigate, or exacerbate, issues associated with spending excessive time in a particular posture.

Generating and outputting a recommendation comprises running a recommendation algorithm configured to:
receive, as an input variable, an initial dental procedure schedule, a predicted posture and optionally a predicted posture duration; and
determine, as output, based on the input variables, a recommended dental procedure schedule.

Recommending a dental procedure schedule can assist a dental professional to minimize the need to continuously maintain the same procedure.

The posture information may comprise information indicating the usage and/or the manner of use and/or the duration of use of at least one item of dental equipment.

The inventors have realized that it is possible to determine the posture of a dental professional using information indicating whether dental equipment is being used (i.e., usage), the way in which the dental equipment is being used (i.e., the orientation of the equipment) and preferably the duration of use. For example, using this information to identify a pattern of usage during a dental procedure may provide insight into the dental professional's posture.

The posture information may indicate that the dental equipment is/is not being used and the manner in which the dental equipment is being used (e.g., the orientation of the dental equipment). The posture information may further indicate the duration over which the dental equipment is used.

The posture information may comprise information:
indicating the usage, manner of use and/or duration of use orientation of a mirror in the dental procedure; and/or
indicating usage, manner of use and/or duration of use of at least one suction tip used in the dental procedure.

The posture information may indicate that the mirror is/is not being used and the manner in which the mirror is being used (e.g., the orientation of the mirror). The posture information may further indicate the duration over which the mirror is used. Additionally, the posture information may indicate that the suction tip is/is not being used and the manner in which the suction tip is being used (e.g., the orientation of the suction tip). The posture information may further indicate the duration over which the suction tip is used.

The posture information may comprise information:
indicating at least one dental instrument used in the dental procedure, and preferably indicating the usage, manner of use and/or duration of use of the dental instrument, and more preferably wherein the posture information comprises information indicating the usage, manner of use and/or duration of a plurality of dental instruments and the sequence in which the dental instruments have been used in the dental procedure; and/or
indicating dental chair and/or patient chair use, duration of use and/or configuration.

According to a further aspect of the invention, there is provided a computer program product comprising code means configured, when run on a processor, to cause the processor to perform a method as described above.

According to a further aspect of the invention, there is provided a system for monitoring dental professional posture, the system comprising:
a posture sensor arrangement comprising at least one sensor for sensing posture information; and
a processor configured to carry out the above-described method steps.

The posture sensor may be a sensor for sensing the usage, manner of usage and/or duration of usage of at least one item of dental equipment or a wearable sensor for sensing the posture of the dental professional.

The system may comprise:
a mirror sensor arranged to output a signal indicating the usage, manner of use and/or duration of use of a mirror; and/or
a suction tip sensor arranged to output a signal indicating the usage, manner of use and/or duration of use of at least one suction tip used in the dental procedure.

The system may comprise both a mirror sensor arranged to detect the manner of use and duration of use the of a mirror and a suction tip sensor arranged to detect the manner of use and duration of use the of a suction tip.

The system may further comprise:
a dental chair sensor arranged to output a signal indicating the usage and/or orientation of a dental chair; and/or
a patient chair sensor arranged to output a signal indicating the usage and/or orientation of a patient chair; and/or
a dental instrument sensor configured to identify at least one dental instrument, and preferably wherein the dental instrument sensor is configured to output a signal indicating the usage, manner of use and/or duration of use of the dental instrument, and more preferably wherein system comprises a plurality of dental instrument sensors arranged to output a signal indicating the usage, manner of use and/or duration of a plurality of dental instruments and the sequence in which the dental instruments have been used in the dental procedure.

The system may comprise both a dental chair sensor arranged to detect the configuration of a dental chair and a patient chair sensor arranged to detect the configuration of the patient chair. That information may be used to determine the distance between the dental chair and the patient chair. The system may further comprise a dental instrument sensor arranged to detect the manner of use and duration of use the of the dental instrument.

In general, the "manner of use" of an item of dental equipment may refer to the orientation/configuration of the dental equipment.

The system may further comprise a display for displaying the posture guidance recommendation.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a flow diagram of a computer implemented method for assessing dental professional posture in accordance with one or more embodiments of the invention;
Fig. 2 is a flow diagram illustrating the use of the method of Fig. 1 to monitor dental professional posture;
Fig. 3 is a flow diagram illustrating a method according to an embodiment in which posture duration is predicted by a prediction algorithm;
Fig. 4 is a schematic diagram illustrating components and processing flow according to one or more embodiments of the invention; and
Fig. 5 is a schematic diagram illustrating a system for assessing dental professional posture according to an embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a computer-implemented method for assessing dental professional posture. The method comprises receiving posture information from a sensor and receiving dental procedure plan information. The dental procedure plan information indicates a dental procedure to be carried out by the dental professional. The method further comprises determining a predicted posture of the dental professional based on the dental procedure plan information. A posture guidance recommendation is generated, based on the posture information and the predicted posture. The posture guidance recommendation is output by the computer.

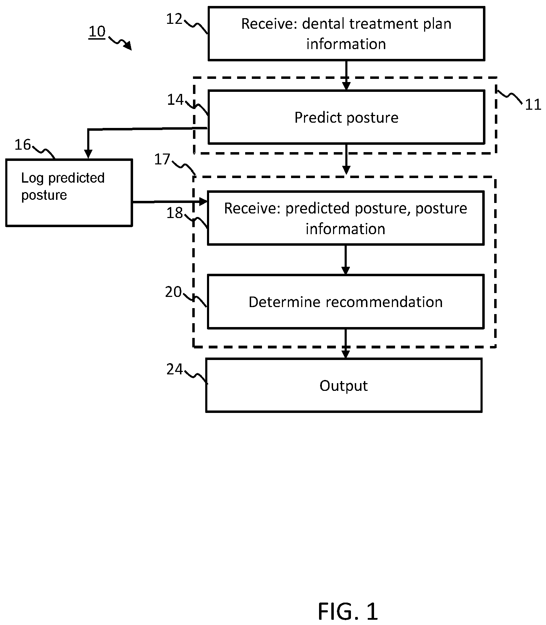

Fig. 1 is flow diagram illustrating a computer implemented method 10 for assessing dental professional posture in accordance with one or more embodiments of the invention. The computer implemented method involves running a prediction algorithm, 11,. The prediction algorithm is configured to predict a posture of a dental professional based on dental procedure plan information input into the prediction algorithm.

The dental procedure plan information may comprise information identifying at least one dental procedure, e.g. a dental treatment, (and optionally information indicating a procedure duration of the dental procedure) or information identifying a series of dental procedures (and optionally the procedure duration associated with each procedure). A dental procedure may be known to be likely to involve a particular posture or a particular combination of postures. Accordingly, by identifying the procedure carried out by the dental professional, using the dental procedure plan information, it is possible to predict dental professional posture.

The computer implemented method 10 also involves running a recommendation algorithm, 17. The recommendation algorithm 17 is a computer trained machine leaning algorithm configured to take, as input, the posture information obtained from at least one sensor and the predicted posture and output a posture guidance recommendation.

The recommendation algorithm 17 may be configured to determine the current posture/previous posture(s) of the dental professional based on the posture information. Using this information together with the predicted posture information (i.e. information indicating a future posture to be adopted by the dental professional), the recommendation algorithm 17 determines a posture guidance recommendation. The posture information may include information indicating both the posture of the dental professional and the time period that the dental professional remained in that posture. This information can be used by the recommendation algorithm to determine the posture guidance recommendation.

The posture information may include information from sensors arranged to directly monitor a dental professional's body position (e.g. using a camera and/or wearable sensors, such as 'smart-clothing' or personal equipment such as headphones/earphones, hearing aids or medical loops, AV/VR glasses) and/or information from sensor arranged to indirectly monitor a dental professional's posture - for example, sensors arranged to monitor of dental equipment (and in particular the use, manner of use and/or duration of use of the dental equipment).

The posture guidance recommendation may comprise, for example, a recommendation to change posture, a recommendation indicating a recommended posture to adopt and/or avoid, a recommended dental treatment compatible with the recommended posture and/or a recommendation indicating a dental treatment to avoid.

As illustrated in Fig. 1, the computer receives, 12, dental procedure plan information and inputs the dental procedure plan information into the prediction algorithm 11 to predict, 14, a future posture of the dental professional.

The dental procedure plan information comprises a treatment schedule indicating a treatment/a series of treatments to be carried out by the dental professional.

Once the predicted posture has been determined, the predicted posture is logged 16.

The logged predicted posture and the posture information are received, 18, as inputs to the recommendation algorithm, which determines, 20, a posture guidance recommendation as output 24.

The posture guidance recommendation may indicate which working positions (postures) would be advisable to adopt in an upcoming period and may further indicate treatment procedures that are compatible with those working positions and/or treatment procedures that are incompatible with those working positions. The posture guidance recommendation may comprise a recommended treatment schedule, in which the procedures to be carried out are scheduled to avoid forcing the dental professional to spend excessive amounts of time in the same posture. The treatment schedule may include combinations of procedures, scheduled breaks between procedures or even during procedures (where appropriate) designed to mitigate risks associated with spending too long in the same posture. For example, the recommendation may include including desk-based dentistry consultations (i.e., teledentistry) with dental treatments carried out in the dental office.

Monitoring dental professional posture and providing posture guidance recommendations in this way may be beneficial for a number of reasons. In particular, assisting dental professionals to monitor their posture in this way can help to improve their working experience, by enabling them to reduce the amount of time spent in the same working position in a convenient way. This can help dental professionals to strike a healthier balance between conducting demanding physical procedures and desk-based work. Patient experience may also be improved by interacting with a relatively well-rested and more comfortable dental professional. It may also result in better health outcomes for patients, since a rested, more comfortable dental professional may be better able to focus on diagnosis and treatment, increasing the chances of correct and effective treatment.

As discussed above, the posture information may be information from sensors for directly or indirectly sensing the dental professional's body position. The inventors have realized that dental professional posture can be predicted based on information indicating the dental equipment used during a dental treatment and/or the manner in which that dental equipment is used (e.g., the orientation/configuration of the dental equipment).

In some embodiments, posture may be predicted using a combination of dental procedure plan information and posture information. That is, posture information (e.g. information indicative of body position, and a duration associated with that body position) may be used in combination with dental procedure plan information (e.g. a dental treatment schedule) to predict the posture adopted by the dental professional.

Additionally, in some embodiments, the method may further comprise determining a posture duration associated with the predicted posture. For example, the posture information and/or dental procedure plan information may be used to determine posture duration associated with the predicted posture. The duration may be determined based on the duration of the treatment to be carried out, according to the dental procedure plan information. Alternatively, the posture duration may be determined using the posture information, which may indicate that the dental professional remained in the same body position for a certain amount of time when carrying out the dental treatment.

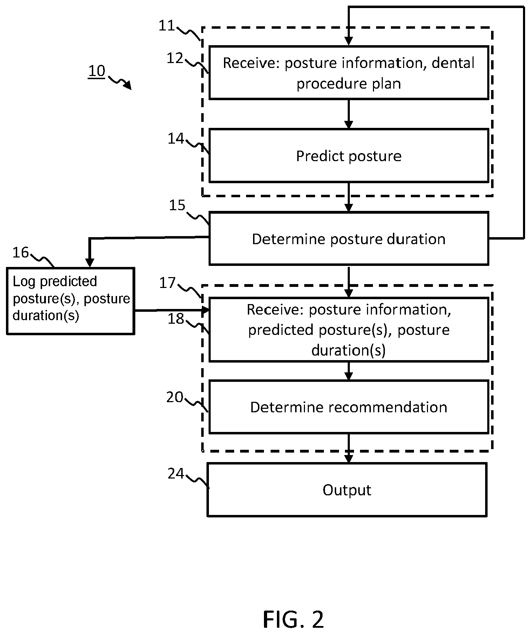

Fig. 2 illustrates an example of a method in which posture is predicted using a combination of posture information and dental procedure plan information. The method illustrated in Fig. 2 additionally includes the step of determining posture duration.

The computer receives, 12, dental procedure plan information and posture information and inputs the information into the prediction algorithm 11 to predict, 14, a future posture of the dental professional.

Next, the computer determines, 12, a posture duration associated with the predicted posture. Once the predicted posture has been determined, the predicted posture and the posture duration are logged 16.

The logged predicted posture, posture duration and the posture information are received, 18, as inputs to the recommendation algorithm, which determines, 20, a posture guidance recommendation as output 24.

Accordingly, in some embodiments, the method comprises receiving dental equipment information and dental procedure plan information and predicting a dental professional's posture based on the received dental equipment information and dental procedure plan information. Predicting the dental professional's posture may comprise determining the posture of the dental professional during an initial time period and using that information, in combination with dental procedure plan information (e.g. information indicating a future dental treatment to be carried out by the dental professional), to predict a future posture of the dental practitioner (e.g. during the future dental treatment to be carried out by the dental professional).

The posture information may be obtained on the same day as the day on which the treatment for which the predicted posture is determined is carried out. In some embodiments, the posture information may be obtained during the same treatment session as the session for which predicted posture is determined.

In the example shown in Fig. 2, the posture information is dental equipment information obtained from sensors arranged to monitor dental equipment.

In particular, information obtained by monitoring dental equipment such as a dental mirror, dental drill, suction equipment, equipment controller (e.g., foot or hand controller), dental instruments (e.g., tools inserted into a patient's mouth), dentist chair and/or patient chair, can be used to assess if and how that equipment is used. For example, information indicating how the dental equipment is used may include the orientation of the equipment (e.g., for the dental mirror, dental instruments, suction equipment), the manner in which dental instruments are gripped by the dental professional (e.g., strength of grip), or the combination of dental equipment being used and/or the sequence of use. The duration of use of the equipment controller can also be used to help predict posture.

Various examples of types of information relating to dental equipment that may be used to predict dental professional posture are described below. It will be appreciated that various combinations of these different types of dental equipment information can be used to predict dental professional posture.

Information relating to use and positioning of a dental mirror can contribute to predicting dental professional posture. Use of a dental mirror during a dental treatment indicates that the dental professional is working on the patient's upper jaw. The orientation of the dental mirror indicates which quadrant the professional is working on. Accordingly, information indicating (i) whether the dental mirror is being used, and (ii) the orientation of the dental mirror can be used to help to identify dental professional posture. The position of the dental mirror can be monitored during treatment using, for example, an inertial measurement unit coupled to the dental mirror.

Information relating to suctioning equipment, used to drain the patient's mouth, can also be used to help predict dental professional posture. In particular, the orientation of suction tips may indicate the location of the treatment site (and the position of the patient relative to the dental professional). Accordingly, information obtained by monitoring suction tip orientation can help to identify dental professional posture.

Information indicating the orientation a dental tool used by a first dental professional (e.g., a dentist) in combination information indicating the orientation of a second dental tool used by a second dental professional (e.g., a dental assistant) can be used to determine dental professional posture.

The orientation of dental equipment (such as a suction tip, mirror, dental instrument) used during a dental procedure, in combination with information indicating the height of the patient's chair and information indicating the height of the dental professional can be used to determine posture.

The position of the dental professional's chair relative to a reference point (e.g., the patient's chair) can also be used to help determine dental professional posture. Accordingly, information indicating the relative position of the dental professional's chair can be used by the prediction algorithm to help determine posture.

The orientation and position (relative to the patient's chair) of a dental tool (e.g., a suction tip, mirror, dental instrument) used during a dental procedure in combination with information relating to the configuration (height, angulation/inclination) of the patient's chair can be used to predict the dental professional posture. The configuration of the dentist's chair may also be used to predict dental professional posture. Information relating to the distance between the patient's chair and the dental professional's chair can also be used to help determine dental professional posture.

Information relating to the configuration of the dental professional's chair and the patient's chair may be obtained from sensors. Alternatively, this information may be obtained from the dental procedure plan. That is, chair orientation may be determined based on the procedure to be carried out.

Information relating to the dental professional's height can also be used to generate the posture guidance recommendation. An unconventional tool orientation is even more detrimental to posture when the dental professional works at a height that is too low or too high.

Further, the sequence in which dental equipment, such as various dental instruments, dental mirrors, suction equipment, dental handpieces, foot controllers, dental chairs and patient chairs, are used during a dental treatment may be used to identify the dental treatment procedure. Accordingly, this information can be used to help determine dental professional posture.

The sequence in which a plurality of dental instruments is used may be indicative of the type of treatment carried out and may be used in combination with dental procedure plan information to determine the posture(s) of the dental professional during the treatment. For example, if the procedure plan indicates that the procedure includes a filling for an upper molar, a root canal treatment for a lower molar and a fitting of a crown on an anterior tooth, it is possible to predict, based on the sequence with which the dental instruments were used, which of the three teeth the dental professional is operating on/in which order the dental professional carried out the treatments. This information can be used to determine dental professional posture.

The distribution of pressure exerted by a dental professional on their chair may can indicate whether the dental professional is sat in a relaxed posture (associated with a substantially equal pressure distribution) or a stressed posture (associated with an unequal pressure distribution). Further, changes in pressure can be indicative of discomfort. For example, a rapid pressure change may indicate that the dental professional is seeking to relieve pain by changing posture. Pressure sensors can be used to monitor the pressure distribution, and the pressure sensor readings can be used as inputs to the prediction algorithm. The pressure sensors may be arranged in different chair legs, in/below the chair cushion or on the wheels of an "office chair".

The duration in which the dental professional maintains the same posture can be predicted based on information relating to the duration over which particular dental instruments are in use. For example, continuous use of a handpiece and/or foot controller over a time period indicates the dental professional has been in the same posture for that time period. Similarly, use of the same dental mirror indicates that the same patient is being treated; therefore, treatment duration can be correlated with the duration of use of the dental mirror.

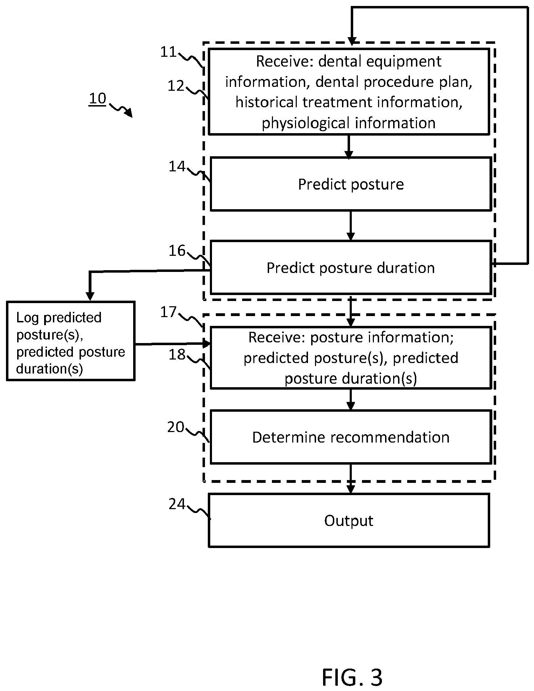

Fig. 3 illustrates another embodiment of the method of Fig. 1, in which the prediction algorithm is further operable to predict posture duration.

In Fig. 3, in addition to the posture information and dental procedure plan, the prediction algorithm receives, 12, historical treatment information and/or physiological information. This information can help to accurately predict the dental professional's posture and/or the duration over which the dental professional is required to maintain that posture.

The historical treatment information may include information indicating how many treatments the dental professional carries out, on average, per day. The physiological information may include patient age/age range, patient height, dental professional height, an indicator of expected patient pain response (e.g., whether the patient copes with pain well, or not) and/or and indicator of expected ease of treatment.

Historical and physiological information may be used to predict posture duration. For example, the average number of procedures conducted per day, the average procedure duration, the expected difficulty of the procedure, patient age and patient pain threshold information may be used to help predict posture duration.

The physiological information may also be used to predict posture. For example, information relating to dental professional height/a height difference between the dental professional and the patient may be used to help determine a predicted posture.

In some embodiments, the method illustrated in Figs. 1-3 comprises predicting a plurality on postures. In these embodiments, the prediction algorithm is operable to determine an initial predicted posture. Based on the initial predicted posture, and the dental procedure plan, the prediction algorithm predicts at least one subsequent posture to be adopted by the dental professional.

For example, a dental procedure may involve a first posture and a second posture. The prediction algorithm may receive information identifying the treatment procedure to be carried out (e.g., from the dental procedure plan) and may predict the posture of the dental professional based on that information (optionally in combination with received posture information). For example, based on the posture information and the dental procedure plan information, the prediction algorithm may predict that the dental professional is in the first posture. Further, the prediction algorithm may predict the next posture to be adopted by the dental professional is the second posture, based on the dental procedure (which is known to involve the first posture followed by the second posture) and the fact that the dental professional is currently determined to be in the first posture.

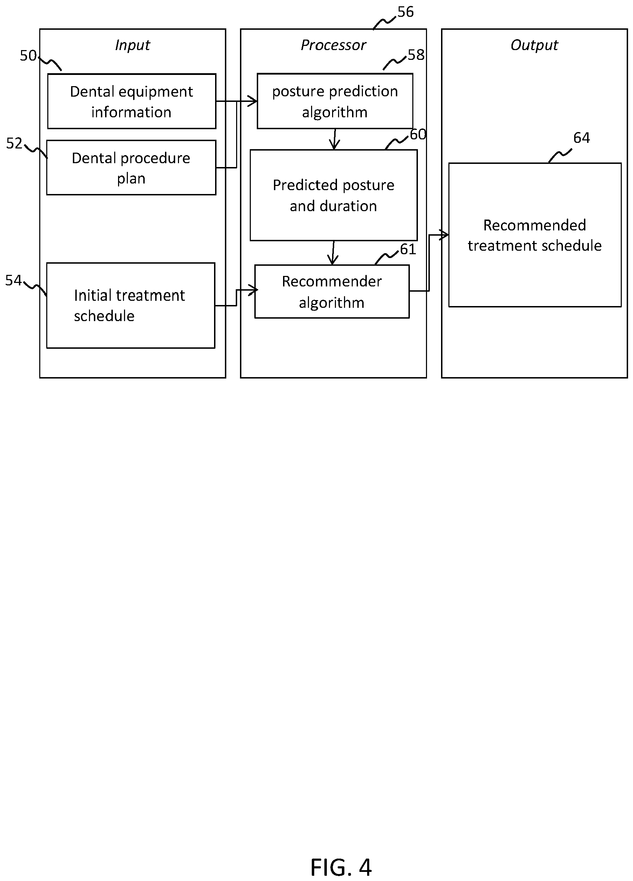

Fig. 4 illustrates the function of a processor 56, according to an embodiment of the invention.

The prediction algorithm 58 receives information on the dental equipment usage/configuration 50, and information relating to a dental procedure plan (e.g., a list of dental treatments to be carried out). This information is obtained from dental equipment sensors, coupled to the dental equipment to monitor usage/configuration.

Based on the inputs 50, 52, the prediction algorithm 58 predicts the dental professional posture and posture duration. The prediction algorithm 58 predicts the posture duration based on the predicted posture and information indicating the treatment to be carried out.

The same or a different algorithm 61 receives as input the predicted posture and posture duration and an initial treatment schedule 54. Then the algorithm 61 produces a recommended treatment schedule 64. The recommended treatment schedule 64 is a modified version of the initial treatment schedule 54 that reduces the amount of time in which the dental professional maintains the same posture. This may be achieved by scheduling treatments involving different postures, to avoid the need for the dental professional to maintain the same posture for extended periods of time. Further, the recommended treatment schedule may recommend breaks between or within dental treatments.

According to some embodiments, instead of outputting a recommended treatment schedule the algorithm 61 may determine a recommended chair position and may produce and output a control signal for actuating an actuator coupled to the dental professional's chair, to move the dental professional's chair towards the recommended chair position.

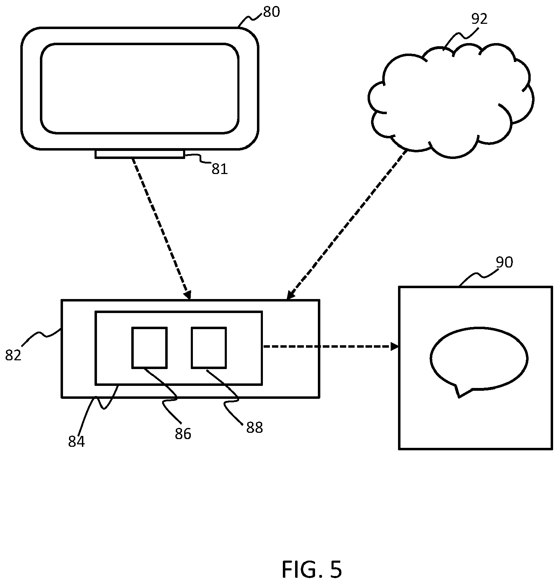

Fig. 5 illustrates a system according to an embodiment of the invention. The system comprises a computer 80 communicatively coupled to a display 90. The computer 82 comprises a processor 84 operable to run a prediction algorithm 86 and a recommendation algorithm 88. The prediction algorithm and the recommendation algorithm may be part of the same algorithm, or they may be separate algorithms. The system further comprises a cloud-based memory comprising a database 92. The database stores a dental procedure plan and historical dental treatment information, such as patient age, average number of patients/treatments performed per day etc.

The system further includes dental equipment, e.g., a dental mirror 80 and a sensor 81 coupled to the dental mirror 82. In use, the processor 84 receives posture information from the sensor 81 indicating the usage and orientation of the dental mirror and dental procedure plan information from the database 92. This information is input to the prediction algorithm 86, which outputs a predicted dental posture. The predicted dental posture and the dental procedure plan are input into the recommendation algorithm 88 which, based on the inputs, determines a posture guidance recommendation. The computer 82 communicates the posture guidance recommendation to the display 90, in the form of a display signal. The display 90 displays the posture guidance recommendation.

It will be appreciated that, in some embodiments, additional information may be used to predict dental posture. For example, dental professional location information may be used as an input for the prediction algorithm.

As noted above, in some embodiments, a part of the method is the use of a prediction algorithm/recommendation algorithm in the form of a trained machine learning algorithm. Details in respect of the trained machine learning algorithm will now be discussed in further detail.

A machine-learning algorithm is any self-training algorithm that processes input data in order to produce or predict output data. In the present case, the input data comprises, at minimum, posture information and dental procedure plan information, and the output data comprises a predicted posture and optionally a predicted posture duration.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines, Naive Bayesian models, and k-nearest neighbor models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g., the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g., ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

In the present case, the training dataset may be based on data contained in a database which comprises data indicating dental professional posture(s) during various dental treatments and information from sensors arranged to directly or indirectly sense body position.

Although in the description above, the various steps of the method are designated as being performed by specific algorithms, it will be appreciated that in other examples, the steps of the method may all be performed by a single algorithm, or by more than two algorithms. The inventive concept is not limited according to any particular division of steps between particular algorithms. The designation is for convenience of description and reflects a most likely implementation in practice. Purely by way of one illustration, the designation of steps recited above to the recommendation algorithm could be broken down further and performed by two separate algorithms, for example one to determine the recommended posture and another to determine the recommended chair position.

As mentioned previously, the invention can be embodied in software form. Thus, another aspect of the invention is a computer program product comprising code means configured, when run on a processor, to cause the processor to perform a method in accordance with example or embodiment of the invention described in this document, or in accordance with any claim of this patent application.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A dental professional may be a dentist or any other type of dental professional such as a dental nurse, a dental assistant, or a dental hygienist.

The posture information may be received from a single sensor or a sensor arrangement comprising multiple sensors.

The dental procedure plan may comprise information indicating dental procedures (e.g., dental treatments) to be carried out by the dental professional.

The dental equipment sensor(s) may be arranged to sense orientation of the dental equipment, such as a drill, dental mirror, suction device. More generally, sensor(s) may be any type of sensor capable of sensing posture information. For example, the sensor(s) may be wearable sensor(s) in the dental professional's clothes, glasses, smartwatch, or headphones/earphones. The sensor(s) may comprise a camera, which is preferably a thermal camera, arranged to monitor posture, vital signs, mental and/or physical stress.

Although Fig. 2 illustrates an embodiment in which the posture information relates to dental equipment, it will be appreciated that in some other embodiments in which posture is predicted using a combination of posture information and dental procedure plan information the posture information does not relate to dental equipment.

Further, although Fig. 2 illustrates a method including the step of determining posture duration, it will be appreciated that in some other embodiments in which posture is predicted using a combination of posture information and dental procedure plan information the method does not include the step of determining posture duration.

While the prediction algorithm and the recommendation algorithm are described as being trained machine learning algorithms, the skilled person will appreciate other types of algorithms could perform the same function(s). Accordingly, in some embodiments one or both of the prediction algorithm and the recommendation algorithm are algorithms other than trained machine learning algorithms.

In embodiments in which the prediction algorithm is a trained machine learning algorithm, the prediction algorithm can be trained using training data including information relating to dental equipment, so that it is trained to predict dental professional posture based on information relating to dental equipment

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A computer-implemented method for assessing dental professional posture, the method comprising:
receiving, from a sensor, posture information;
receiving dental procedure plan information, the dental procedure plan information indicating a dental procedure to be carried out by the dental professional;
determining a predicted posture based on the dental procedure plan information; and
generating and outputting a posture guidance recommendation based on the posture information and the predicted posture.

2. The computer implemented method of claim 1, further comprising determining a posture duration associated with the predicted posture, wherein the posture guidance recommendation is further based on the posture duration.

3. The computer implemented method of claim 1 or claim 2, wherein determining a predicted posture comprises:
running a prediction algorithm configured to:
receive, as input variables, posture information and dental procedure plan information identifying a procedure to be performed by the dental professional, and
determine, as output, based on the input variables, a predicted posture, and preferably wherein the prediction algorithm is further configured to determine, as output, a posture duration.

4. The computer implemented method of claim 3 further comprising, receiving, as an input variable to the prediction algorithm, physiological information and/or historical procedure information.

5. The computer implemented method of any preceding claim further comprising predicting, based on the predicted posture and the dental procedure plan information, a subsequent predicted posture, wherein predicting the subsequent predicted posture comprises:
running a prediction algorithm configured to:
receive, as an input variable, an initial predicted posture and a procedure identifier, and
determine, as output, based on the input variables, a subsequent predicted posture.

6. The computer implemented method of claim 5, further comprising predicting, based on the dental procedure plan information, a subsequent posture duration associated with the subsequent predicted posture, wherein the prediction algorithm is configured to:
receive, as input variables, an initial predicted posture and a procedure identifier, and
determine, as output, based on the input variables, a subsequent posture duration.

7. The computer implemented method of any preceding claim wherein generating and outputting a posture guidance recommendation comprises running a recommendation algorithm configured to:
receive, as input variables, the posture information, the predicted posture and posture duration, and
determine, as output, based on the input variables, a recommended posture or a recommendation to avoid a posture,
and optionally wherein the method further comprises determining, as output, at least one dental procedure compatible with the recommended posture or at least one dental procedure associated with the posture it is recommended to avoid.

8. The computer implemented method of any preceding claim, wherein generating and outputting a recommendation comprises running a recommendation algorithm configured to:
receive, as an input variable, an initial dental procedure schedule, a predicted posture and optionally a predicted posture duration; and
determine, as output, based on the input variables, a recommended dental procedure schedule.

9. The computer implemented method of any preceding claim, wherein the posture information comprises information indicating the usage and/or the manner of use and/or the duration of use of at least one item of dental equipment.

10. The computer implemented method of any preceding claim, wherein the posture information comprises information:
indicating the usage, manner of use and/or duration of use orientation of a mirror in the dental procedure; and/or
indicating usage, manner of use and/or duration of use of at least one suction tip used in the dental procedure.

11. The computer implemented method of any preceding claim wherein the posture information comprises information:
indicating at least one dental instrument used in the dental procedure, and preferably indicating the usage, manner of use and/or duration of use of the dental instrument, and more preferably wherein the posture information comprises information indicating the usage, manner of use and/or duration of a plurality of dental instruments and the sequence in which the dental instruments have been used in the dental procedure; and/or
indicating dental chair and/or patient chair use, duration of use and/or configuration.

12. A computer program product comprising code means configured, when run on a processor, to cause the processor to perform a method in accordance with any one of claims 1 to 11.

13. A system for monitoring dental professional posture, the system comprising:
a posture sensor arrangement comprising at least one sensor for sensing posture information; and
a processor configured to carry out the steps of any of claims 1 to 11.

14. The system of claim 13, wherein system comprises:
a mirror sensor arranged to output a signal indicating the usage, manner of use and/or duration of use of a mirror; and/or
a suction tip sensor arranged to output a signal indicating the usage, manner of use and/or duration of use of at least one suction tip used in the dental procedure.

15. The system of claim 13 or claim 14, further comprising:
a dental chair sensor arranged to output a signal indicating the usage and/or orientation of a dental chair; and/or
a patient chair sensor arranged to output a signal indicating the usage and/or orientation of a patient chair; and/or
a dental instrument sensor configured to identify at least one dental instrument, and preferably wherein the dental instrument sensor is configured to output a signal indicating the usage, manner of use and/or duration of use of the dental instrument, and more preferably wherein system comprises a plurality of dental instrument sensors arranged to output a signal indicating the usage, manner of use and/or duration of a plurality of dental instruments and the sequence in which the dental instruments have been used in the dental procedure.
